# EUROPEAN PATENT APPLICATION

(11) **EP 3 476 249 A1**
(43) Date of publication of application: **01.05.2019**
(21) Application number: 18193081.9
(22) Date of filing: 07.09.2018
(51) Int. Cl.: A46B 9/04, A46B 5/02, A46B 17/06, A61L 2/10, B08B 7/00

(54) **ORAL CARE IMPLEMENT STERILIZATION SYSTEM**

(30) Priority: 07.09.2017 CN 201710804248
(71) Applicant: Colgate-Palmolive Company, New York, NY 10022 (US)
(72) Inventor: XIE, Fan Gang, Shanghai, 200125 (CN); YOU, Changxin, Shanghai (CN)
(74) Representative: Schicker, Silvia

(57) **Abstract**

An oral care sterilization system in one embodiment includes an oral care implement and sterilization unit. The oral care implement may be a toothbrush including tooth cleaning elements on a head portion and a first coupling member. The sterilization unit includes a housing and second coupling member that mutually engages the first coupling member to secure the toothbrush to the sterilization unit. Magnetic coupling may be used in one embodiment. The toothbrush may be mounted in a suspended manner from the sterilization unit. A sterilization element such as an ultraviolet light source disposed in the housing irradiates at least the toothbrush head portion to destroy bacterial contamination. In one aspect, the toothbrush and housing each include complementary thematic features sharing a common motif. The motif is of a nature to make brushing and care of the toothbrush more interesting for younger users.

## Description

### BACKGROUND

The present invention relates to oral care systems, and particularly to a sterilization system for sterilizing oral care implements

Oral care implements such as toothbrushes are susceptible to oral bacteria contamination resulting from normal hygienic use and handling. Bacterial accumulations may be especially prevalent on the head portion of the toothbrush, particularly within the tooth cleaning elements such as bristles and/or elastomeric cleaning elements. The bacteria can contribute to tooth decay and gum disease. It is desirable to decontaminate or sterilize the toothbrush between brushings to minimize or eliminate the bacterial contamination. This becomes especially problematic for young users such as children or adolescents who may lack the motivation or interest to adhere to a proper brushing regimen and care of their toothbrushes.

### BRIEF SUMMARY

The present invention provides an oral care sterilization system that is designed to disinfect an oral care implement such as a toothbrush and entice younger users to adhere to a brushing and sterilization regimen. In one embodiment, the system may generally include a combination of an electrically-powered sterilization unit and toothbrush. The sterilization unit includes a docking station configured to removably couple and support the toothbrush in proximity to a sterilizing element. The sterilizing element is operable to kill bacteria that may accumulate during brushing particularly on the head area of the toothbrush body, which contains the tooth cleaning elements. In one implementation, the sterilizing element may be an ultraviolet (UV) light source, which emits UV radiation in the disinfection spectrum (UV-C). In one embodiment, the toothbrush may be vertically suspended from the sterilization unit during sterilization.

The oral care sterilization system may further include thematic features sharing a common motif to make using the system interesting and fun especially for younger users (i.e. children/adolescents). Any type of motif may be used, including for example without limitation themes/motifs such as extraterrestrials, outer space, superheroes, cartoon characters, military, sports, music, art, or others especially popular with younger users. In one embodiment, the sterilization unit and toothbrush form a kit that may include complementary thematic features including shapes and/or indicia (e.g. words, numbers, images, etc.) embodying the common motif, as further described herein. The thematic features need not be identical on the toothbrush and sterilization unit, but preferably are complementary in nature and share the common motif.

In one embodiment, the invention may be an oral care sterilization system comprises: an oral care implement including an elongated body having a plurality of tooth cleaning elements and a first coupling member, the oral care implement having a first thematic feature; a sterilization unit including a housing and second coupling member configured for removable attachment of the oral care implement to the sterilization unit, the housing having a second thematic feature; and a sterilization element disposed in the housing proximate to the oral care implement when mounted therein, the sterilization element arranged and operable to irradiate a head portion of the oral care implement with ultraviolet radiation; wherein the first and second thematic features are complementary and share a common motif.

In another embodiment, the invention may be an oral care kit comprising: a toothbrush including an elongated body comprising a longitudinal axis, a distal head portion having a plurality of tooth cleaning elements, a proximal handle portion, and a first magnetic coupling member, the toothbrush including a first thematic feature; a disk-shaped sterilization unit including a housing defining a vertical axis, a downwardly open internal cavity, an ultraviolet light source disposed in the cavity, and a second magnetic coupling member disposed in the cavity, the housing including a second thematic feature; the toothbrush is removably suspended from the sterilization unit via releasable engagement between the first and second magnetic coupling members; wherein the ultraviolet light source is arranged and operable to irradiate at least the head portion of the oral care implement with ultraviolet radiation; and wherein the first and second thematic features are complementary and share a common motif.

In another embodiment, the invention may be a method for sterilizing an oral care implement. The method comprises: positioning a toothbrush below a bottom opening of an ultraviolet sterilization unit; raising the toothbrush vertically upwards towards the bottom opening; inserting a head portion of the toothbrush into an internal cavity of the sterilization unit; and engaging a first coupling member on the toothbrush with a second coupling member in the sterilization unit; wherein the sterilization unit and toothbrush include complementary thematic features having a common motif.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### BRIEF DESCRIPTION OF THE DRAWINGS

The present invention will become more fully understood from the detailed description and the accompanying drawings, wherein:
FIG. 1 is a perspective view of an oral care sterilization system according to one embodiment of the present invention including an oral care implement in the form of a toothbrush and sterilization unit;
FIG. 2 is a perspective view of the toothbrush of FIG. 1;
FIG. 3 is a front view thereof;
FIG. 4 is a side view thereof;
FIG. 5 is a rear view thereof;
FIG. 6 is an enlarged detail of the head portion of the toothbrush;
FIG. 7 is an enlarged detail of the handle portion of the toothbrush;
FIG. 8 is a bottom perspective view showing the toothbrush docked in the sterilization unit;
FIG. 9 is a top view of the sterilization unit;
FIG. 10 is a bottom view thereof;
FIG. 11 is a side view thereof;
FIG. 12 is a side cross-sectional view thereof;
FIG. 13 is a bottom view thereof having an alternative embodiment of a coupling member for mounting the toothbrush to the sterilization unit;
FIG. 14 is a side cross-sectional view thereof;
FIG. 15 is bottom view of the sterilization unit having an alternative embodiment of an ultraviolet light source of annular configuration;
FIG. 16 is a side cross-sectional view thereof;
FIG. 17 is side view of a mounting stand for supporting the sterilization unit;
FIG. 18 is a side view of an alternative embodiment of the mounting stand;
FIG. 19 is a bottom view of an alternative embodiment of the sterilization unit configured with an electric plug for direct insertion into an electrical wall outlet or socket; and
FIG. 20 is a side view of an alternative embodiment of the sterilization unit.
All drawing are schematic and not necessarily to scale.

### DETAILED DESCRIPTION

The following description of the preferred embodiment(s) is merely exemplary in nature and is in no way intended to limit the invention, its application, or uses.

In the description of embodiments disclosed herein, any reference to direction or orientation is merely intended for convenience of description and is not intended in any way to limit the scope of the present invention. Relative terms such as "lower," "upper," "horizontal," "vertical," "above," "below," "up," "down," "top" and "bottom" as well as derivatives thereof (e.g., "horizontally," "downwardly," "upwardly," etc.) should be construed to refer to the orientation as then described or as shown in the drawing under discussion. These relative terms are for convenience of description only and do not require that the apparatus be constructed or operated in a particular orientation unless explicitly indicated as such. Terms such as "attached," "affixed," "connected," "coupled," "interconnected," and similar refer to a relationship wherein structures are secured or attached to one another either directly or indirectly through intervening structures, as well as both movable or rigid attachments or relationships, unless expressly described otherwise.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

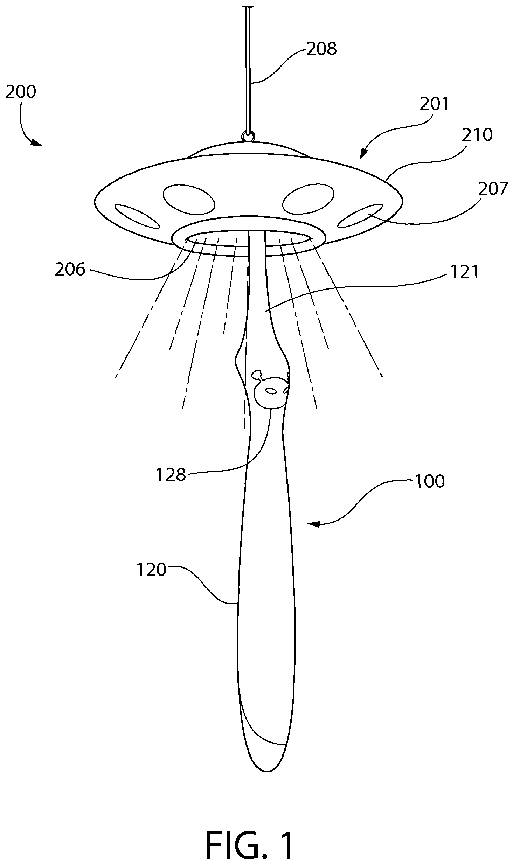

Referring to FIG. 1, a non-limiting embodiment of an oral care sterilization system 200 according to the present disclosure is shown. The system generally includes a sterilization unit 201 and an oral care implement, which may be a toothbrush 100 in one non-limiting embodiment. The sterilization unit 201 includes a docking station 220, which allows a user to removably and releasably secure the toothbrush 100 to the unit for disinfection, as further described herein.

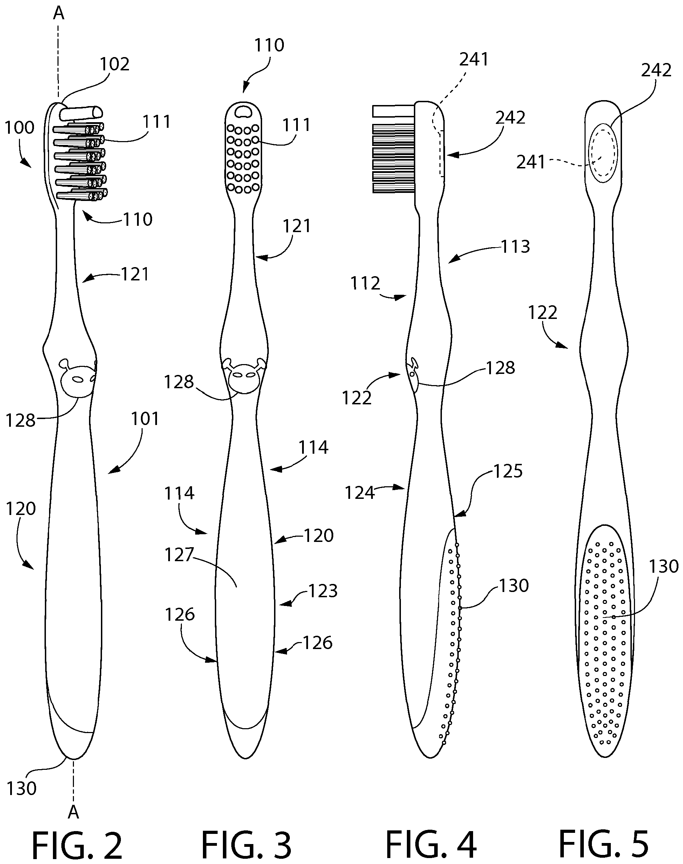

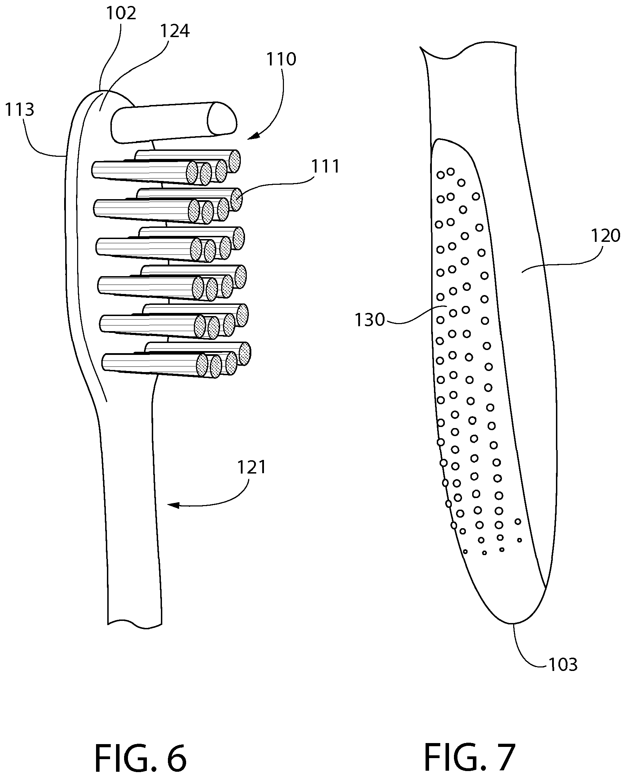

Referring now to FIGS. 2-7, toothbrush 100 generally includes an elongated body 101 extending from a proximal end 103 to a distal end 102 along a longitudinal axis A-A. The body 101 includes a front side 112, opposing rear side 113, and pair of opposing lateral sides 114 extending between the front and rear sides. The longitudinal axis A-A follows the contours and shapes of the toothbrush body 101 from proximal to distal ends 103, 102 and remains at the centerline of each transverse section of the body through which the longitudinal axis extends. Accordingly, the longitudinal axis A-A is not necessarily a straight reference line in all cases depending on the shape and curvature of the toothbrush body.

Body 101 further comprises a head portion 110, a handle portion 120, and a neck portion 121 coupling the handle to head. In certain embodiments, neck portion 121 may be a structure that is narrower in width (measured transversely to longitudinal axis A-A) than the head portion 110 and shorter in length than the handle portion 120 for grasping by a user.

The front side 112 of the head portion 110 may be substantially planar in one embodiment. The head portion 110 comprises a plurality of tooth cleaning elements 111 extending transversely from the front side 112. The exact types, structure, pattern, orientation and material of the tooth cleaning elements 111 is not limiting of the present invention unless so specified in the claims. As used herein, the term "tooth cleaning elements" is used in a generic sense to refer to any structure or combination of structures that can be used to clean, polish or wipe the teeth and/or soft oral tissue (e.g. tongue, cheek, gums, etc.) through relative surface contact. Common examples of "tooth cleaning elements" include, without limitation, bristle tufts, filament bristles, fiber bristles, nylon bristles, spiral bristles, rubber bristles, elastomeric protrusions, flexible polymer protrusions, combinations thereof and/or structures containing such materials or combinations. Suitable elastomeric materials include any biocompatible resilient material suitable for uses in an oral hygiene apparatus. To provide optimum comfort as well as cleaning benefits, the elastomeric material of the tooth or soft tissue engaging elements may have a hardness property in the range of A8 to A25 Shore hardness. One suitable elastomeric material is styrene-ethylene/butylene-styrene block copolymer (SEBS) manufactured by GLS Corporation. Nevertheless, SEBS material from other manufacturers or other materials within and outside the noted hardness range could be used.

The tooth cleaning elements 111 of the present invention can be connected to the head portion 110 in any manner now available or to be developed and is also not limiting of the invention. For example, staples/anchors, in-mold tufting (IMT) or anchor free tufting (AFT) could be used to mount the cleaning elements/tooth engaging elements. In AFT, a plate or membrane is secured to the brush head such as by ultrasonic welding. The bristles extend through the plate or membrane. The free ends of the bristles on one side of the plate or membrane perform the cleaning function. The ends of the bristles on the other side of the plate or membrane are melted together by heat to be anchored in place. Any suitable form of cleaning elements may be used in the broad practice of this invention. Alternatively, the bristles could be mounted to tuft blocks or sections by extending through suitable openings in the tuft blocks so that the base of the bristles is mounted within or below the tuft block.

In certain embodiments, the head portion 110 may also include a soft tissue cleanser (not shown herein) coupled to or positioned on its rear side 113. An example of a suitable soft tissue cleanser that may be used with the present invention and positioned on the rear surface of the head portion 110 is disclosed in U.S. Patent No. 7,143,462, issued December 5, 2006 to the assignee of the present application, the entirety of which is hereby incorporated by reference. In certain embodiments, the soft tissue cleanser may include a plurality of protuberances, which can take the form of elongated ridges, nubs, or combinations thereof. Of course, the invention is not to be so limited and in certain embodiments the oral care implement 100 may not include any soft tissue cleanser.

In the exemplified embodiment, the head portion 110 is formed integrally with the handle portion 120 and neck portion 121 as a single unitary structure using a molding, milling, machining, and/or other suitable process. However, in other embodiments the handle portion 120, neck portion 121, and head portion 110 may be formed as separate components which are operably connected at a later stage of the manufacturing process by any suitable technique known in the art, including without limitation thermal or ultrasonic welding, a tight-fit assembly, a coupling sleeve, threaded engagement, adhesion, or fasteners. In certain embodiments, the head and neck portions 110, 121 may be formed as a detachable single unitary structure which is configured for removable coupling to the handle portion 120, thereby allowing the head to be replaceable when the tooth cleaning elements 111 have worn.

With continuing reference to FIGS. 2-7, the handle portion 120 is an axially elongated structure extending from the proximal end 103 of the body 101 to the neck portion 121 that provides a means for grasping and manipulating the toothbrush 100 during use. The handle portion 120 may comprise an ergonomically contoured thumb grip section 122 adjoining the neck portion 121 and a finger grip section 123 adjoining the thumb grip section that is disposed more proximally. The thumb grip section 122 is located between the neck portion 121 and the finger grip section 123. Handle portion 120 further defines a front surface 124, an opposing rear surface 125, and two opposing lateral side surfaces 126. Surfaces 124-126 collectively form an outer surface 127 of the handle portion 120.

In the exemplified embodiment, the handle portion 120 is generically depicted having various contours for user comfort. More specifically, in the exemplified embodiment the thumb grip section 122 of the handle portion 120 is a more bulbous diametrically enlarged structure relative to the outer surface 127 of and other portions of the handle portion 120. Thus, thumb grip section 122 may have a diameter and width measured transversely to longitudinal axis A-A between lateral side surfaces 126 of the handle portion 120 that is greater than a width of the finger grip section 123 of the handle portion. Of course, the invention is not to be so limited in all embodiments, and in certain other embodiments the thumb grip section 122 may not have a greater width than the entire or at least portions of the finger grip section 123. For example, the proximal portion of the finger grip section 123 may be bulbous shaped and wider than other portions of the finger grip section in addition to or instead of the thumb grip section 122. The handle portion 120 can therefore take on a wide variety of shapes, contours and configurations, none of which are limiting of the present invention unless so specified in the claims.

In the exemplified embodiment, the toothbrush body 101 may be made of a substantially rigid plastic material, such as for example without limitation polymers and copolymers of ethylene, propylene, butadiene, vinyl compounds and polyesters such as polyethylene terephthalate. Of course, the invention is not to be so limited in all embodiments and the handle portion 120 may be formed with a semi-rigid material.

Handle portion 120 may further include various surface portions which are formed of a non-slip resiliently flexible material for greater comfort and handling, such as without limitation a thermoplastic elastomer (TPE) affixed over portions of or the entirety of the handle portion 120 to enhance grip control of the toothbrush during use. For example, parts of the handle portion 120 that are typically gripped by a user's palm, fingers, and/or thumb during use, such as the finger grip section 123 and thumb grip section 122, may be partially or totally overmolded with a thermoplastic elastomer or other resilient material to further increase comfort and grip for a user. The TPE may be overmolded onto the more rigid plastic used to form the body 101 of toothbrush 100 during the fabrication process.

In the illustrated embodiment, for example, a finger grip control member 130 formed of the foregoing resilient material may be disposed on parts of the rear and side surfaces 125, 126 of the handle portion 120 of toothbrush 100. In one embodiment, thumb grip section 122 of the toothbrush handle portion 120 may include a thumb grip control member 128 formed of the resilient material to facilitate gripping the toothbrush. Grip control member 128 may be disposed on the front surface 124 of the thumb grip section, as shown.

The sterilization unit 201 of the oral care sterilization system 200 will now be further described with initial reference to FIGS. 1 and 8-12. Sterilization unit 201 includes a partially hollow housing 210 including a top surface 202, bottom surface 203, and peripheral sides 204 therebetween that define a peripheral edge and perimeter of the housing. A downwardly open cavity 206 formed in the bottom surface 203 of the housing is configured for insertably receiving at least a part of the body 101 of toothbrush 100 therein, as further described herein. Housing 210 defines a vertical centerline or axis VA extending through the geometric centerline of the housing.

Housing 210 is preferably made of any suitable light-weight material. In one embodiment, the housing is made of suitable substantially rigid plastic material such as for example without limitation polymers and copolymers of ethylene, propylene, butadiene, vinyl compounds and polyesters such as polyethylene terephthalate. Of course, the invention is not to be so limited in all embodiments and the housing 210 may be formed of other suitable rigid materials or a combination of different types of materials.

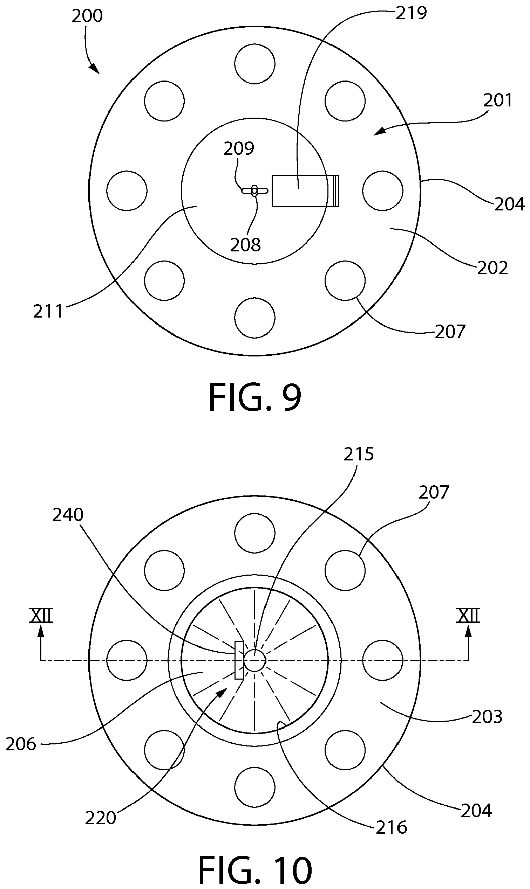

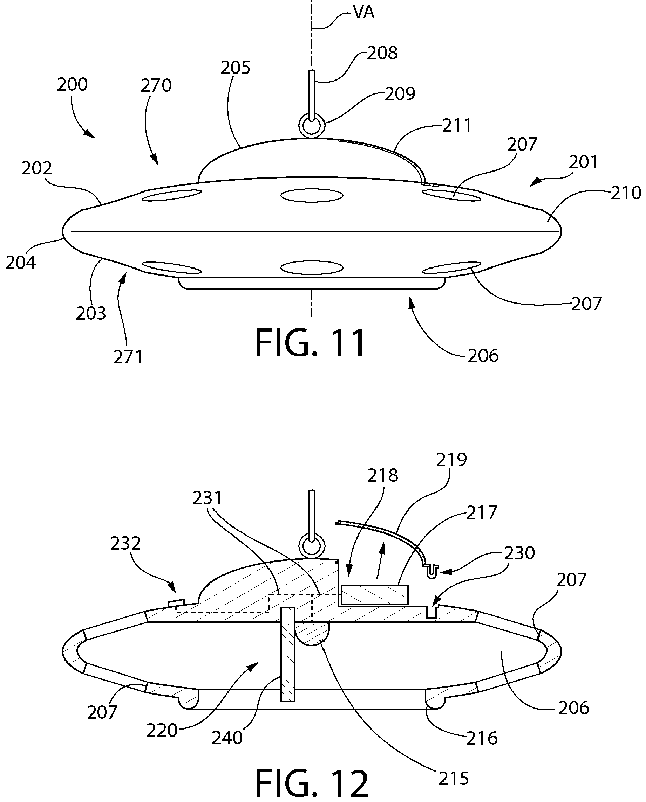

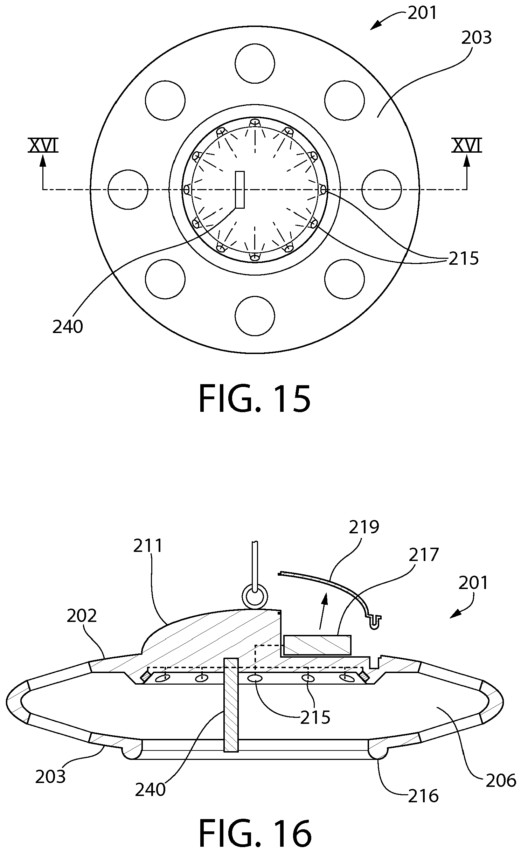

To provide the sterilization functionality, the sterilization unit 201 further includes a sterilization element such as an ultraviolet (UV) light source 215 configured and operable to irradiate the toothbrush 100 with ultraviolet radiation in the disinfection spectrum (i.e. UV-C) suitable to kill bacterial accumulations on at least the head portion 110 of the toothbrush body. UV light source 215 is disposed in cavity 206 and accessible via bottom opening 216 formed in the bottom surface 203 of the housing 210 Any suitable commercially-available UV-C light source may be used, such as for example without limitation least one UV light emitting diode (LED), a UV xenon bulb, or a UV mercury bulb. In one embodiment, a single UV light bulb or LED may be provided if sufficient UV radiation can be generated to disinfect the toothbrush 100 (see, e.g. FIGS. 10 and 12). The single UV source may be disposed above the toothbrush head portion 120 when attached to the sterilization unit 201. Lateral placement of the single bulb or LED may also be used. In yet other embodiments, an array of UV light bulbs or LEDs may be provided (see, e.g. FIGS. 15 and 16) which are arranged above and/or laterally around the toothbrush head portion 120 to irradiate the head from a plurality of sides. The array of UV bulbs or LEDs may be arranged in an annular pattern extending a full 360 degrees around the head portion 120 of the toothbrush in certain implementations as shown. In other embodiments, the UV array may extend in an arc less than 360 degrees around the toothbrush.

Light source 215 is powered by a suitable electrical power source. The power source may be an on-board power source such as disposable or rechargeable battery or batteries 217. The batteries may be disposed in a battery compartment 218 formed in a portion of the sterilization unit housing 210. In one embodiment, battery compartment 218 may be disposed in the top portion of housing 210 and include a removable battery compartment cover 219. Cover 219 may be secured to housing 210 by a locking mechanism 230, which may comprise a resilient tab on the cover which engages a mating slot formed in the housing (as shown) that provides a snap-lock. Other locking mechanisms may of course be used to secure the cover to the housing. The cover 219 may be arcuately curved in one embodiment to conform to the shape and contour of the portion of the housing where the battery compartment 218 is located. Battery compartment 218 includes the usual electrical contacts to electrically conduct electrical current from the batteries 217 to the UV light source 215 via wiring 231 run through the housing 210. An on/off power switch 232 is electrically connected and wired to the power source (battery compartment 218) for turning the UV light source 215 on or off. The switch 232 may be mounted at any suitable location on the sterilization unit housing 210.

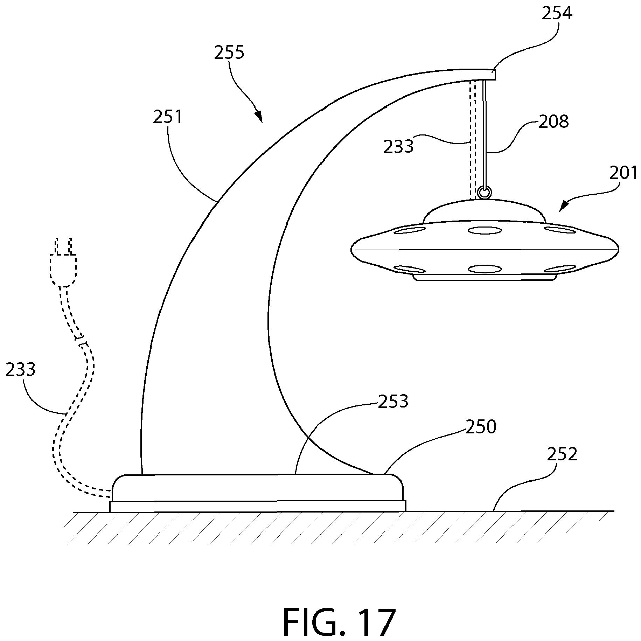

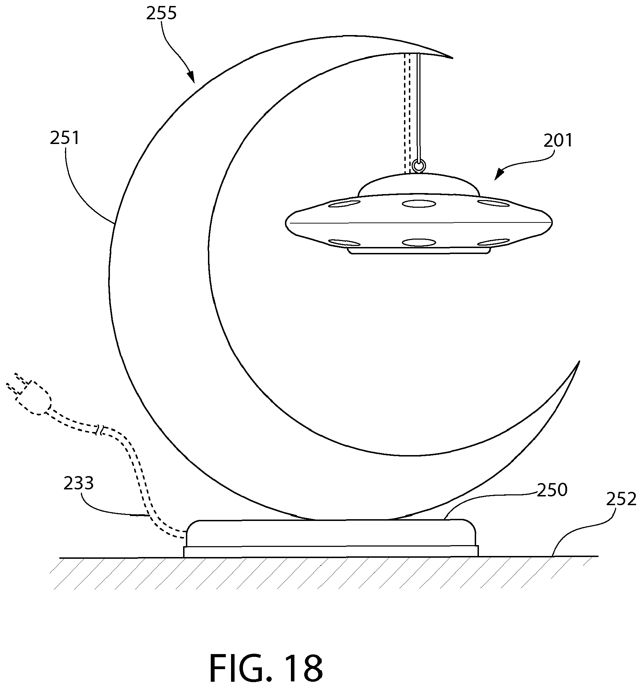

In other embodiments, the electrical power source may also be a more permanent type power source such as a conventional electric utility power grid supply that provides standard AC line voltage (e.g. 120V or 220V) to the UV light source 215. An electric cord (wire conductor) 233 having a standard wall outlet electric plug on one end carries the electric current to the UV light source 215 in the sterilization unit 201 (see, e.g. FIGS. 17 and 18). A step-down transformer may be provided if required to convert the AC line voltage to DC low voltage power usable by the light source 215. Such a transformer may be either directly plugged into an available electric wall socket or incorporated in the housing 210 of the sterilization unit 201.

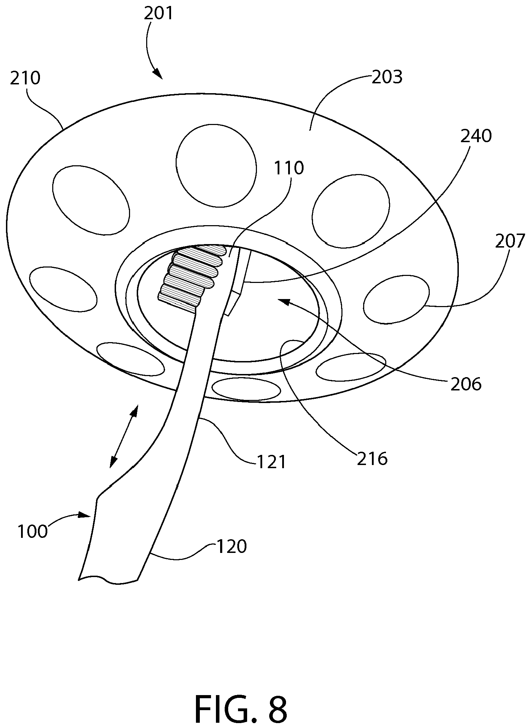

The sterilization unit 201 includes a docking station 220 that provides a coupling mechanism configured for releasably attaching and holding the toothbrush 100 in the unit while the toothbrush 100 is undergoing sterilization and for storage thereafter. Referring to FIGS. 8-12, the coupling mechanism may be magnetic in one non-limiting implementation. Docking station 220 in this embodiment includes a magnet 240 which is mounted in cavity 206 of the sterilization unit housing 210. The magnet forms a first coupling member. Magnet 240 is accessible through the bottom opening 216 formed in the bottom surface 203 of the housing 210. The magnet may be mounted proximate to vertical axis VA of the sterilization unit 201 near the center of the cavity 206 as shown, but slightly offset to one side. This allows the toothbrush 100 to be located along and parallel to the vertical axis VA when mounted in the sterilization unit. Magnet 240 is further located proximate to UV light source 215 to allow the toothbrush 100 to be properly irradiated with UV radiation from the light source.

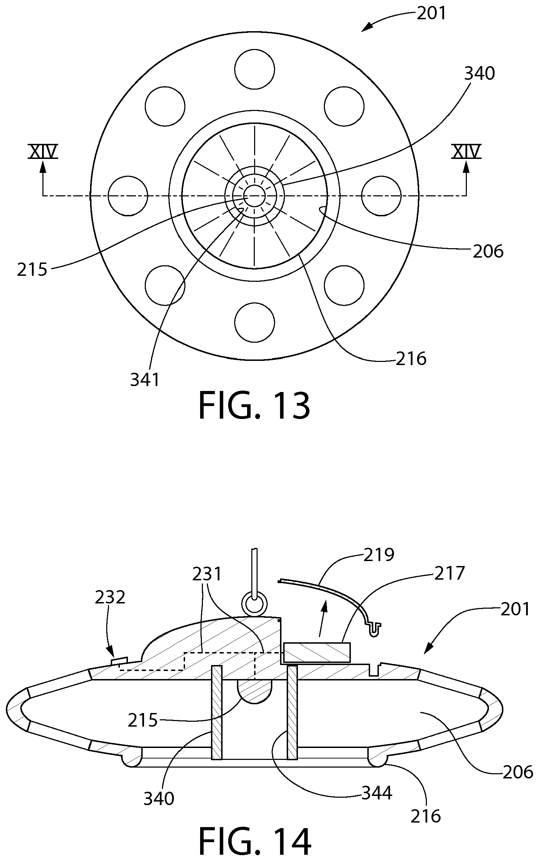

In one embodiment, magnet 240 may be a generally plate-like and flat structure in configuration having a generally rectilinear shape (e.g. rectangular or square). Other polygonal and non-polygonal (e.g. circular, etc.) shapes be used. In alternate embodiments, magnet 240 may have a ring-like annular configuration such as annular magnet 340 shown in FIGS. 13 and 14. Magnet 340 defines a central receptacle 341 configured to receive the head portion 110 of toothbrush 100 therein. The UV light source 215 is mounted at the top of the receptacle 341 for irradiating the toothbrush. The annular magnet 341 requires less precise placement and insertion of the toothbrush head into the receptacle and engagement with the magnet. The toothbrush 100 may have any rotational orientation therefore when coupled to the annular magnet 340. This contrasts to use of the flat magnet 240 in which the toothbrush has a single rotational orientation when properly coupled thereto for sterilization. It will be appreciated that other shapes of magnets may be used. Any suitable type of commercially-available magnet may be used.

The second coupling member of the toothbrush-to-docking station magnetic coupling mechanism is formed by a magnetic metal element 241 disposed on the toothbrush 100. The magnetic metal element 241 may be formed of any suitable ferro- and ferrimagnetic material capable of being magnetically attracted and coupled to magnet 240 in the sterilization unit docking station 220. Steel may be used in one non-limiting example; however, other magnetic metals attracted to a magnet by magnetic force may be used. In alternative embodiment, a second magnet may be substituted for magnetic metal.

In one embodiment, the magnetic metal element 241 is disposed in the head portion 110 of the toothbrush body 101. This ensures that when the toothbrush 100 is mounted to magnet 240 in the sterilization unit 210, the head portion 110 is positioned proximate to the UV light source 215 for optimum sterilization. In one configuration, the magnetic metal element 241 may comprise a thin metal plate of suitable configuration, which is attached to the rear side 113 of the toothbrush head portion 110 opposite front side 112 containing the tooth cleaning elements. To avoid direct contact of the metal plate with the toothbrush user's mouth, the magnetic metal element 241 may be recessed below the rear surface of the rear side 113 of toothbrush head portion 110 and covered by an overlying material. In one embodiment, the overlying material 242 may be an elastomeric material as already described herein that is overmolded on the head portion 110 of the toothbrush 100. The overlying material is preferably thin and just thick enough to cover the magnetic metal element 241 while still allowing secure magnetic coupling of the toothbrush to the magnet 240 of the sterilization unit docking station 220. Other types of overlying materials may of course be used.

A method for sterilizing a toothbrush 100 using UV sterilization unit 201 will now be briefly described. The user first positions the toothbrush 100 below the bottom opening 206 of the sterilization unit 201 and rotationally orients the toothbrush 100 so that the rear side 113 of the toothbrush head portion 110 with magnetic metal element 241 faces the docking station magnet 240 (i.e. tooth cleaning elements 111 on front side 112 of the toothbrush facing away from the magnet). The user then raises and vertically inserts the head portion 110 of the toothbrush 100 upwards through the bottom opening 216 into the internal cavity 206 of the sterilization unit housing 210. The user next engages the rear side 113 of the toothbrush head portion 110 with the magnet 240, which magnetically couples the toothbrush thereto due to the magnetic attraction between the magnet and magnetic metal element 241 disposed in the toothbrush head. Notably, toothbrush 100 is supported in a dangling or suspended manner from the sterilization unit 201 by the head portion 110 as shown in which the toothbrush handle portion 120 does not engage a support surface for support. Support for the toothbrush is provided solely by the magnetic coupling mechanism in this embodiment. Toothbrush 100 is held in a substantially upright and vertical position, with some angular deviation being possible depending on the accuracy of the user emplacing the toothbrush in the sterilization unit. With the toothbrush mounted in the sterilization unit, the user may then activate the UV light source 215 to sterilize the head portion 110 toothbrush via operating the on/off power switch 232. A timer circuit may be provided to activate the UV light source 215 for a predetermined period of time necessary to sterilize the toothbrush. The timer circuit is electrically coupled to the UV light source 215 power supply and operable to disconnect power from the light source once the timer times out. UV light source 215 may stay on continuously during the sterilization period or be pulsed in some embodiments.

When the toothbrush 100 is coupled to the docking station 220 of the sterilization unit 201, a majority of the length of the toothbrush body 101 may remain exposed and extend below the sterilization unit housing 210 as seen in FIG. 1. In one arrangement, the entire handle portion 120 and part of the neck portion 121 remain below the bottom surface 203 of the housing. It is most important that at least the head portion 110 and immediately adjoining neck portion 121 which are typically inserted into the user's mouth when brushing are subjected to irradiation by the UV light source 215 and sterilized. Accordingly, the housing 210 need only have a sufficient height in some embodiments to receive the head and adjoining neck portions of the therein for sterilization.

In the present embodiment being described, it bears noting that longitudinal axis A-A of toothbrush 100 is coaxially aligned with the vertical axis VA of the sterilization unit 201. In other embodiments, the toothbrush 100 may be mounted off-axis with respect to vertical axis VA. Accordingly, the invention is not limited to a centered toothbrush coupling arrangement to the sterilization unit.

In the foregoing embodiment described, it bears noting that the magnet 240 is mounted in the sterilization unit 201 and the magnetic metal element 241 is mounted in the toothbrush 100. However, it will be appreciated by those skilled in the art that this arrangement may be reversed in other embodiments to provide the magnetic coupling mechanism.

Although a magnetic coupling mechanism is described herein for removably coupling the toothbrush 100 to the sterilization unit 201, other types of coupling may be used such as mechanical couplings including snap lock mechanisms, hangers, or other. Accordingly, the invention is expressly not limited to magnetic coupling mechanisms alone.

Referring now to FIGS. 1, 11-12, 18-19, the sterilization unit 201 in one embodiment is configured to be hung in a freely suspended manner from an available overhead support. Housing 210 may therefore include a mounting assembly comprising a coupler such as eye hook 209 mounted to the top surface 202 of the housing 210 and a hanger 208 connected thereto. The hanger 208 may be a flexible wire, string, or similar in some implementations as shown. A more rigid metal wire or plastic o hanger may also be used in other embodiments. To maintain the housing 210 in level orientation, the hook 209 and connected hanger 208 are preferably coaxially mounted to and aligned with vertical axis VA of the sterilization unit 201 as shown.

In some embodiments, the overhead support may a ceiling of a room. When such a mounting arrangement is not possible or desired, the overhead support may be provided by a mounting stand 255 comprising a base 250 and attached cantilevered support arm 251 as shown in FIGS. 17 and 18. Base 250 has a flat bottom configured to rest on an available horizontal support surface 252, such as the counter of a bathroom vanity or cabinet. Arm 251 has portions which extend vertically upwards from the base and laterally outwards to provide clearance for the sterilization unit housing 210. Arm 251 includes a bottom end 253 attached to base 250 and a terminal free end 254 to which the hanger 208 of the sterilization unit housing 210 is connected. Base 250 has a sufficient width to provide stable mounting for the sterilization unit 201. In embodiments where AC house line voltage is used to power the UV light source 215 as described above, the electric cord 233 may be routed through the mounting stand to the sterilization unit 201 as shown. In other embodiments where available counter space may be limited, the base 250 may be omitted and the arm 251 may be configured for direct attachment to an available vertical wall such as via suitable fasteners.

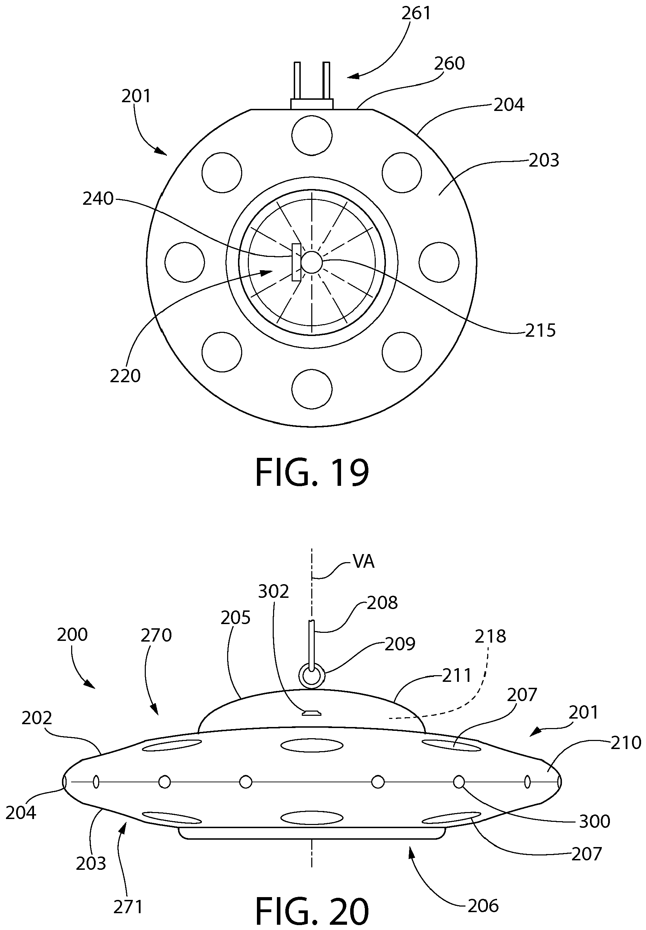

In an alternative embodiment, the sterilization unit 201 may be configured to be plugged in directly to an available electric wall outlet or socket where AC house line voltage is used to power the UV light source 215. Such a configuration is shown for example in FIG. 19. The housing 210 of the sterilization unit 201 may include a flat peripheral surface 260 which includes an integrated electric plug 261 with standard prong arrangement to plug the unit into the wall socket (not shown). The sterilization unit 201 is therefore hung in a cantilevered manner from the wall outlet. Based on the foregoing embodiments, it will be appreciated that numerous mounting variations are possible for sterilization unit 201 and does not limit the invention.

According to another aspect of the invention, the oral care sterilization system 200 may have a motif that is intended to make the brushing and sterilization experience more fun for younger users. This will motivate these users to adhere to a regular oral care regimen. This is achieved by configuring and/or adorning the toothbrush 100 and sterilization unit 201 with thematic features sharing a common motif. This combines the concepts of playing and brushing for younger users.

A non-limiting example of an oral care sterilization system having thematic features sharing a common motif is illustrated herein. The motif is extraterrestrials in this case. The sterilization unit 201 has thematic features which are indicative of a UFO (unidentified flying object). The housing 210 has a disk-shaped configuration that resembles a "flying saucer" and thus embodies a thematic feature by configuration. The housing an upper portion 270 and lower portion 271 separated by the peripheral sides 204 or edge. The upper and lower portions may be arcuately curved and convex shaped as shown in one embodiment. A raised dome 211 is disposed on the upper portion 270, which may conveniently house the battery compartment, controls, wiring, UV light source, and other related functional appurtenances already described above as best shown in FIG. 12. The eye hook 209 for hanger 208 may also be mounted to the dome structure. The lower portion 271 and part of the upper portion 270 define the internal cavity 206 of the housing 210 and may therefore be substantially hollow.

To further exemplify the extraterrestrial motif, a plurality of open ports 207 may be formed through the lower portion 271 and/or upper portion 270 of the sterilization unit housing 210 thereby providing an additional thematic feature. When the UV light source 215 is activated, the bluish-purple light emitted will glow and shine through the ports 207, in addition to through the bottom opening 216. This contributes to replicating an appearance commonly associated with UFOs. In some embodiments, the light source 215 may be pulsed for effect rather than having a continuous intensity. The ports 207 may be physically open structures in some embodiments, or in other embodiments may include a transparent or translucent window to partially obscure the internal structure of the housing 210. It will be appreciated that in addition to providing the primary function of sterilizing the toothbrush 100, the sterilization unit 201 further serves as a night light which is beneficial for younger users.

In addition to the structural features and configuration of the sterilization unit housing 210 described above which serve as thematic features of the common extraterrestrial motif, housing 210 may further include markings or indicia that further exemplify and comprise the motif. The indicia may be formed by appliques or paint applied to the surface of the housing 210, or indicia which are integrally formed with and/or embedded via molding and overmolding of the housing. Such indicia may include for example without limitation numerical and alphabetic characters forming words and numbers, graphic or photographic images and designs, or other markings evincing the extraterrestrial motif. Any types of indicia or markings may be therefore be used.

The toothbrush 100 also includes thematic features sharing the common extraterrestrial motif in this example, which complement the thematic features of the sterilization unit housing 210 described above. Referring to FIGS. 1-4, the toothbrush thematic features may include an indicia 128 in the form of an extraterrestrial being image applied or embedded in the body 101 of the toothbrush 100. The image in this non-limiting example is the head of an extraterrestrial being. In one embodiment, the indicia image may be formed of a resilient elastomeric material overmolded onto the body of the oral care implement and positioned to provide a non-slip gripping surface for the user. The gripping surface may be a thumb grip positioned on the distal-most portion of the toothbrush handle portion 120 adjacent to the neck portion 121. It will be appreciated that various other indicia and positioning on the toothbrush body may be used instead of or in addition to indicia 128, which demonstrates just one non-limiting example.

It bears noting that indicia 128 of toothbrush 100 representing a first thematic feature is not identical to the set of second thematic features of the sterilization unit 201 described above comprising the UFO. However, the first and second thematic features still share the common extraterrestrial motif and are therefore complementary in nature.

When the toothbrush 100 is magnetically docked in the sterilization unit 201 as shown in FIG. 1, the common extraterrestrial motif is apparent. Of interest to younger users, the indicia 128 on the toothbrush handle portion 120 is illuminated by UV light emitted through the bottom opening 216 of the sterilization unit housing 210 which contributes to a positive and fun brushing experience.

If a mounting stand 255 is to be used and provided, the stand may include a third thematic feature sharing the common extraterrestrial motif with the toothbrush and sterilization unit. An example is shown in FIG. 18 depicting a mounting stand 255 having a support arm 251 configured as the moon that defines a thematic feature by configuration. Various other typeos of thematic features such as indicia may also be provided in other embodiments.

It will be appreciated that numerous other common motifs may be used for the sterilization unit 201 and toothbrush 100; examples of which have already been described herein. Accordingly, the invention is not limited to the extraterrestrial motif described herein for illustrative purposes only.

FIG. 20 depicts an alternative embodiment of a sterilization unit 201A. This embodiment includes a circumferential array of decorative LEDs 300 arranged in an annular pattern or ring along the lateral sides 204 of the sterilization unit housing 210. The LEDs 300 may be powered by the same power source used to power the UV light source 215 (i.e. battery(ies) or building AC/DC line current). This embodiment illustrates a USB port 302 that can be used to supply transformed AC/DC low voltage power to the UV light source. In this case, power cord 233 may have a male USB plug which is inserted into the female USB port. USB port may be a regular size or mini-USB type port. Alternatively, USB port 302 may be used to recharge a lithium ion battery if used instead as the power source and is electrically connected to the battery compartment 218. In other embodiments, the USB power port 302 may alternatively be used to separately power the ring of decorative LEDs 300 separate from powering the UV light source 215. In other embodiments, the decorative LEDs 300 may be arranged or emplaced in different locations on the housing 210 of the sterilization unit 201.

While the invention has been described with respect to specific examples including presently preferred modes of carrying out the invention, those skilled in the art will appreciate that there are numerous variations and permutations of the above described systems and techniques. It is to be understood that other embodiments may be utilized and structural and functional modifications may be made without departing from the scope of the present invention. Thus, the spirit and scope of the invention should be construed broadly as set forth in the appended claims.

## Claims

1. An oral care sterilization system comprising:
an oral care implement including an elongated body having a plurality of tooth cleaning elements and a first thematic feature;
a sterilization unit including a housing configured for removable attachment of the oral care implement to the sterilization unit, the sterilization unit having a second thematic feature; and
a sterilization element disposed in the housing proximate to the oral care implement when attached thereto, the sterilization element arranged and operable to irradiate a head portion of the oral care implement with ultraviolet radiation;
wherein the first and second thematic features are complementary and share a common motif.

2. The oral care sterilization system according to claim 1, wherein the oral care implement is vertically suspended from the sterilization unit when mounted therein.

3. The oral care sterilization system according to claims 1 or 2, wherein the housing includes a shape that defines the second thematic feature and the first thematic feature is an indicia disposed on a body of the oral care implement having a complementary motif to the housing shape, optionally wherein the housing is shaped like a flying saucer and the indicia on the oral care implement is an image of an extraterrestrial being.

4. The oral care sterilization system according to claim 3 wherein the indicia on the oral care implement is formed of a resilient elastomeric material overmolded on the body of the oral care implement providing a non-slip gripping surface.

5. The oral care sterilization system according to any of claims 1 to 4, wherein the oral care implement is magnetically attached to the housing of the sterilization unit, optionally wherein the oral care implement has a magnetic metal element and the housing of the sterilization unit has a magnet that magnetically and releasably secures the oral care implement to the housing.

6. The oral care sterilization system according to any one of claims 1 to 5, wherein the housing defines a downwardly open cavity and a first coupling member, and a head portion of the oral care implement includes a second coupling member, the oral care implement being insertable into the cavity to engage the first and second coupling members for securing the oral care implement to the sterilization unit.

7. The oral care sterilization system according to any one of claims 1 to 6, wherein the sterilization element is an ultraviolet light source comprising at least one light emitting diode, a xenon bulb, or a mercury bulb, and further comprising a power source comprised of one or more batteries removably disposed in the housing, the power source configured to provide electric power to the light source.

8. The oral care sterilization system according to any one of claims 1 to 7, wherein the housing of the sterilization unit is hung in a suspended manner from a support.

9. The oral care sterilization system according to any one of claims 1 to 8, wherein the housing of the sterilization unit includes an integral electrical plug configured for insertion into an electrical wall socket.

10. An oral care kit comprising:
a toothbrush including an elongated body comprising a longitudinal axis, a distal head portion having a plurality of tooth cleaning elements, a proximal handle portion, the head portion comprising a first coupling member;
a sterilization unit including a housing defining a vertical axis, a downwardly open internal cavity, an ultraviolet light source disposed in the cavity, and a second coupling member disposed in the cavity;
the toothbrush removably suspended from the sterilization unit via releasable engagement between the first and second coupling members;
wherein the ultraviolet light source is arranged and operable to irradiate at least the head portion of the oral care implement with ultraviolet radiation.

11. The oral care kit according to claim 10, wherein the sterilization unit is hung in a suspended manner from an overhead support.

12. The oral care kit according to any one of claims 10 to 11, wherein the housing has a shape defining a flying saucer that defines a first thematic feature and the toothbrush has an indicia of an extraterrestrial alien that defines a second thematic feature, the first and second thematic features defining a common motif that is extraterrestrials, optionally wherein the indicia on the toothbrush is formed of a resilient elastomeric material overmolded on the body of the toothbrush positioned to provide a non-slip thumb gripping surface.

13. The oral care kit according to any one of claims 10 to 12, wherein the first coupling member on the toothbrush is a magnetic metal element and the second coupling member of the sterilization unit is a magnet that magnetically and releasably secures the toothbrush to the sterilization unit, optionally wherein the magnetic metal element is recessed in the body of the toothbrush and covered by a thin layer of an overlying elastomeric material overmolded onto the body of the toothbrush.

14. A method for sterilizing an oral care implement, the method comprising:
positioning a toothbrush below a bottom opening of an ultraviolet sterilization unit;
raising the toothbrush vertically upwards towards the bottom opening;
inserting a head portion of the toothbrush through the bottom opening and into an internal cavity of the sterilization unit; and
engaging a first coupling member on the toothbrush with a second coupling member in the sterilization unit.

15. The method according to claim 14, wherein the toothbrush is hung from the sterilization unit in a suspended manner.
